(19) Europäisches Patentamt
European Patent Office
Office européen des brevets

(11) Veröffentlichungsnummer: 0 330 645 A2

(12) # EUROPÄISCHE PATENTANMELDUNG

(21) Anmeldenummer: 89890011.3

(22) Anmeldetag: 13.01.89

(51) Int. Cl.4: C 07 C 103/34
C 07 D 295/18,
C 07 C 121/38,
C 07 C 103/167,
A 61 K 31/16, A 61 K 31/445,
A 61 K 31/275

(30) Priorität: 23.02.88 AT 437/88

(43) Veröffentlichungstag der Anmeldung: 30.08.89 Patentblatt 89/35

(84) Benannte Vertragsstaaten: BE CH DE ES FR GB GR IT LI LU NL SE

(71) Anmelder: Gerot-Pharmazeutika Gesellschaft m.b.H.
Arnethgasse 3
A-1171 Wien (AT)

(72) Erfinder: Schlager, Ludwig H., Dr.
Nottebohmstrasse 12
A-1190 Wien (AT)

(74) Vertreter: Pawloy, Heinrich, Dr. et al
Riemergasse 14
A-1010 Wien (AT)

(54) **Neue aromatische Aldehyde, ihre Herstellung und Verwendung.**

㊼ Es sind neue aromatische Aldehyde der allgemeinen Formel

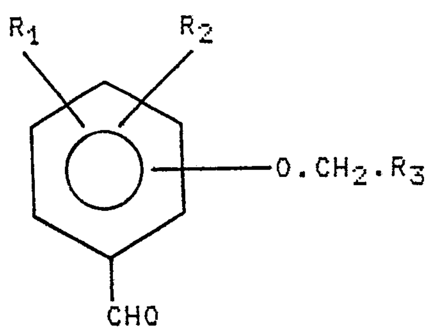

(I),

worin $R_1$ und $R_2$ jeweils Wasserstoff, Halogen, Nitro oder Methoxy bedeuten und $R_3$ entweder eine Cyangruppe oder die Gruppierung $-CO.NR_4R_5$ darstellt, wobei $R_4$ und $R_5$ die in der Beschreibung angegebenen Bedeutungen haben, und ein Verfahren zu deren Herstellung geoffenbart.

Die neuen Aldehyde sind als Zwischenprodukte zur Herstellung von pharmazeutischen Wirkstoffen oder Agrochemikalien geeignet.

EP 0 330 645 A2

**Beschreibung**

**Neue aromatische Aldehyde, ihre Herstellung und Verwendung**

Die Erfindung betrifft neue aromatische Aldehyde der allgemeinen Formel

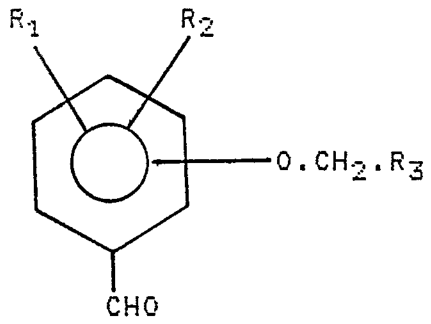

(I),

worin $R_1$ and $R_2$ jeweils Wasserstoff, Halogen, Nitro oder Methoxy bedeuten und $R_3$ entweder eine Cyangruppe oder die Gruppierung $-CO.NR_4R_5$ darstellt, wobei $R_4$ und $R_5$ jeweils entweder Wasserstoff oder ein niederer Alkylrest mit 1 bis 6 C-Atomen sein können oder worin $R_4$ zusammen mit $R_5$ einen gesättigten 5- oder 6-gliedrigen Heteroring bildet, der noch als weiteres Heteroatom Sauerstoff, Stickstoff oder Schwefel enthalten kann, mit der Maßgabe, daß der Rest $-O.CH_2.R_3$ nicht in Stellung 4 steht, wenn $R_1$ und/oder $R_2$ Wasserstoff und/oder Methoxy bedeuten, und daß im Falle von $R_1 = R_2 = R_4 = R_5 =$ Wasserstoff oder für den Fall $R_1 = H$, $R_2 = 3\text{-}OCH_3$ und $R_4 = R_5 =$ Äthyl der Carbamylmethoxyrest nicht in Stellung 2 steht, sowie mit der weiteren Maßgabe, daß im Falle von $R_1 = R_2 = Cl$ in den Stellungen 3 und 5 der Rest $R_3$ in Stellung 2 nicht CN oder $CONH_2$ bedeuten kann.

Die neuen aromatischen Aldehyde der obigen Formel (I) können als Zwischenprodukte zur Herstellung von pharmazeutischen Wirkstoffen, z.B. von Calcium-Antagonisten (vgl. AT-Patentanmeldung A 3306/87) oder Agrochemikalien verwendet werden.

Zur Herstellung der erfindungsgemäßen Aldehyde setzt man einen Hydroxybenzaldehyd der allgemeinen Formel

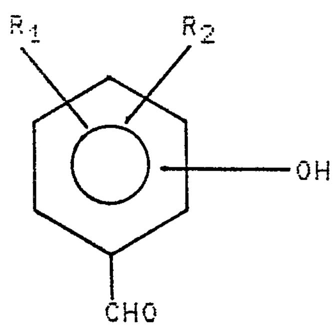

(II),

worin $R_1$ und $R_2$ die oben genannte Bedeutung haben, oder dessen Alkaliphenolat vorzugsweise in einem inerten Lösungsmittel oder Lösungsmittelgemisch und gegebenenfalls in Anwesenheit eines Phasentransfer-Katalysators mit einer Verbindung der allgemeinen Formel

$X.CH_2R_3$ (III),

worin $R_3$ die obige Bedeutung hat und X einen als HX abspaltbaren Rest, wie z.B. ein Halogenatom oder einen Sulfonyloxy-Rest, darstellt, um.

Als Hydroxybenzaldehyde der allgemeinen Formel (II) eignen sich z.B. Salicylaldehyd, Vanillin, ortho-Vanillin, 5-Bromsalicylaldehyd, 5-Chlorsalicylaldehyd, 3-Hydroxybenzaldehyd, 5-Nitro-vanillin und dgl.

Die nachfolgenden Beispiele sollen die Erfindung erläutern, ohne sie jedoch darauf zu beschränken. Temperaturangaben beziehen sich jeweils auf Celsiusgrade.

**Beispiel 1:**

Zu einer Lösung von 5,5 g 3,5-Dichlorsalicylaldehyd in 80 ml absol. n-Propanol tropft man unter Rühren 5,19 g einer 30 %igen Lösung von Natriummethylat in Methanol, fügt dann 7,73 g 1-Chloracetyl-4-(2-methoxy-phenyl)-piperazin (vgl.: J.Med. Chem. 11, 1147 (1968)) zu und erhitzt das Gemisch 8 h unter Rückfluß. Danach dampft man die Mischung am Rotationsverdampfer ein, nimmt den Rückstand in Methanol auf und filtriert. Der

Eindampfrückstand des Filtrats kristallisiert aus Äthylacetat/Isopropyläther. Der erhaltene 3,5-Dichlor-2-[4-(2-methoxy-phenyl)-piperazino-carbonylmethoxy]-benzaldehyd schmilzt bei 121 - 123°.

Wird die gleiche Reaktion mit Salicylaldehyd durchgeführt, dann entsteht der entsprechende 2-[4-(2-Methoxy-phenyl)-piperazino-carbonylmethoxy]-benzaldehyd nur in öliger Form.

**Beispiel 2:**

Eine Lösung von 20 g Salicylaldehyd in 300 ml Methanol wird unter Rühren mit 29,5 g einer 30 %igen Lösung von Natriummethylat in Methanol versetzt und dann am Rotationsverdampfer eingedampft. Der Rückstand wird nach Zusatz von 1 g Triäthylbenzylammoniumchlorid in 100 ml Chloracetonitril 2 h bei 55° gerührt. Durch Eindampfen der Mischung erhält man überschüssiges Chloracetonitril zurück. Der Rückstand wird in Äthylacetat aufgenommen, die Lösung mit Wasser ausgeschüttelt, die organische Phase mit $Na_2SO_4$ siccum getrocknet und eingedampft. Der Rückstand kristallisiert beim Stehen. Aus Isopropyläther umkristallisiert schmilzt der erhaltene 2-Cyanmethoxybenzaldehyd bei 78 - 82°.

**Beispiel 3:**

Man tropft zu einer unter Rückfluß gerührten Lösung von 49,3 g Salicylaldehyd und 66 g N-(2-Chloracetyl)-morpholin in 400 ml absol. n-Propanol langsam 72,7 g einer 30 %igen Lösung von Natriummethylat in Methanol. Nach weiteren 4 h Rückfluß dampft man das Gemisch am Rotationsverdampfer ein, nimmt den Rückstand in Äthylacetat auf und schüttelt mit Wasser aus. Nach dem Trocknen der organischen Phase mit $Na_2SO_4$ siccum dampft man diese wieder ein. Der ölige Rückstand wird beim Stehen kristallin. Nach dem Verrühren mit Isopropyläther, Absaugen und Trocknen erhält man den bei 108° schmelzenden 2-(4-Morpholinocarbonylmethoxy)-benzaldehyd.

Weitere neue Derivate der allgemeinen Formel (I), die nach dem erfindungsgemäßen Verfahren erhalten werden, sind in der folgenden Tabelle angeführt:

Derivate gemäß Formel (I):

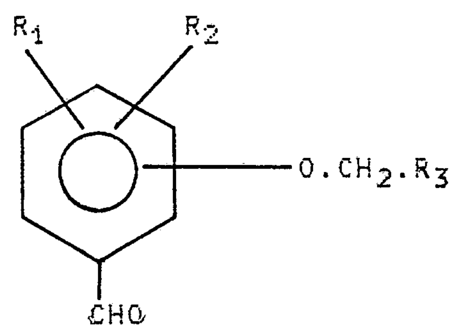

| $R_1$ | $R_2$ | Stellung des $OCH_2R_3$-Restes | $R_3$ | Fp.°C (Kp°C/Pa) |
|---|---|---|---|---|
| H | H | 2 | $CON(CH_3)_2$ | 84 - 86 |
| H | 3-$OCH_3$ | 2 | $CON(CH_3)_2$ | öl (180/133) |
| H | H | 2 | $CON(C_2H_5)_2$ | 39 - 41 |
| H | H | 3 | $CON(CH_3)_2$ | öl (161-162/266) |
| H | H | 3 | $CON(C_2H_5)_2$ | öl (147-151/133) |
| H | H | 2 | CO.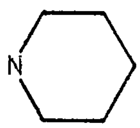 | 67 - 69 |
| H | H | 3 | CN | öl (121-122/133) |
| 2-$NO_2$ | H | 5 | $CON(C_2H_5)_2$ | 109-111 |
| 3-$OCH_3$ | 5-$NO_2$ | 4 | CN | 96 - 98 |
| H | 5-$NO_2$ | 2 | CN | 118 - 121 |
| 2-$NO_2$ | H | 5 | CN | 80 - 88 |
| H | 5-Br | 2 | CN | 105 - 108 |
| H | 5-Cl | 2 | CN | 92 - 95 |

**Patentansprüche**

1. Neue aromatische Aldehyde der allgemeinen Formel

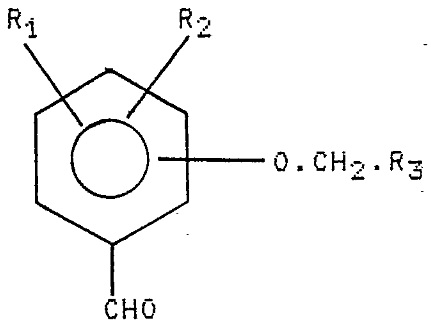 (I),

worin $R_1$ und $R_2$ jeweils Wasserstoff, Halogen, Nitro oder Methoxy bedeuten und $R_3$ entweder eine Cyangruppe oder die Gruppierung $-CO.NR_4R_5$ darstellt, wobei $R_4$ und $R_5$ jeweils entweder Wasserstoff oder ein niederer Alkylrest mit 1 bis 6 C-Atomen sein können oder worin $R_4$ zusammen mit $R_5$ einen gesättigten 5- oder 6-gliedrigen Heteroring bildet, der noch als weiteres Heteroatom Sauerstoff, Stickstoff oder Schwefel enthalten kann, mit der Maßgabe, daß der Rest $-O.CH_2.R_3$ nicht in Stellung 4 steht, wenn $R_1$ und/oder $R_2$ Wasserstoff und/oder Methoxy bedeuten, und daß im Falle von $R_1 = R_2 = R_4 = R_5 =$ Wasserstoff oder für den Fall $R_1 = H$, $R_2 = 3\text{-}OCH_3$ und $R_4 = R_5 =$ Äthyl der Carbamylmethoxyrest nicht in Stellung 2 steht, sowie mit der weiteren Maßgabe, daß im Falle von $R_1 = R_2 = Cl$ in den Stellungen 3 und 5 der Rest $R_3$ in Stellung 2 nicht CN oder $CONH_2$ bedeuten kann.

2. Verfahren zur Herstellung neuer aromatischer Aldehyde der allgemeinen Formel (I), wie in Anspruch 1 definiert, dadurch gekennzeichnet, daß man Hydroxybenzaldehyde der allgemeinen Formel

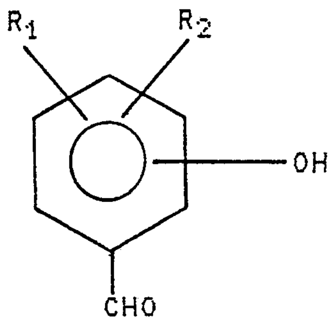 (II),

worin $R_1$ und $R_2$ die in Anspruch 1 angegebene Bedeutung haben, oder deren Alkaliphenolate vorzugsweise in einem inerten Lösungsmittel oder Lösungsmittelgemisch und gegebenenfalls in Anwesenheit eines Phasentransfer-Katalysators mit einer Verbindung der allgemeinen Formel

$X.CH_2R_3$ (III),

worin $R_3$ die obige Bedeutung hat und X einen als HX abspaltbaren Rest, wie z.B. ein Halogenatom oder einen Sulfonyloxy-Rest, darstellt, umsetzt.

3. Verwendung neuer aromatischer Aldehyde der allgemeinen Formel (I) als Zwischenprodukte zur Herstellung von pharmazeutischen Wirkstoffen, z.B. von Calcium-Antagonisten, oder Agrochemikalien.